(19) Europäisches Patentamt
European Patent Office
Office européen des brevets

(11) Veröffentlichungsnummer: **0 251 082**
**B1**

# (12) EUROPÄISCHE PATENTSCHRIFT

(45) Veröffentlichungstag der Patentschrift: **23.05.90**

(21) Anmeldenummer: **87108846.4**

(22) Anmeldetag: **20.06.87**

(51) Int. Cl.$^5$: **C07C 69/734,** C07C 69/736, C07C 323/22, A01N 37/10, A01N 37/36, A01N 37/38

## (54) Substituierte Acrylsäureester und Fungizide, die diese Verbindungen enthalten.

(30) Priorität: **21.06.86 DE 3620860**

(43) Veröffentlichungstag der Anmeldung: **07.01.88 Patentblatt 88/1**

(45) Bekanntmachung des Hinweises auf die Patenterteilung: **23.05.90 Patentblatt 90/21**

(84) Benannte Vertragsstaaten: **AT BE CH DE ES FR GB GR IT LI NL SE**

(56) Entgegenhaltungen:
**EP-A- 0 178 826**
**EP-A- 0 203 606**

(73) Patentinhaber: **BASF Aktiengesellschaft, Carl-Bosch-Strasse 38, D-6700 Ludwigshafen(DE)**

(72) Erfinder: **Karbach, Stefan, Dr., Sperlinggasse 3, D-6700 Ludwigshafen(DE)**
Erfinder: **Schirmer, Ulrich, Dr., Berghalde 79, D-6900 Heidelberg(DE)**
Erfinder: **Rentzea, Costin, Dr., Richard-Kuhn Strasse 1-3, D-6900 Heidelberg(DE)**
Erfinder: **Pommer, Ernst-Heinrich, Dr., Berliner Platz 7, D-6703 Limburgerhof(DE)**
Erfinder: **Ammermann, Eberhard, Dr., Sachsenstrasse 3, D-6700 Ludwigshafen(DE)**
Erfinder: **Steglich, Wolfgang, Prof. Dr., Hobsweg 77, D-5300 Bonn-Roettgen(DE)**
Erfinder: **Schwalge, Barbara Agnes Maria, Dr., Tannenweg 9, D-5204 Lohmar 1(DE)**
Erfinder: **Anke, Timm, Prof. Dr., Theodor Heuss Strasse 17, D-6750 Kaiserslautern(DE)**

EP 0 251 082 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue substituierte Acrylsäurederivate und Fungizide, welche diese Verbindungen enthalten.

Es ist bekannt, N-Tridecyl-2,6-dimethylmorpholin oder seine Salze, z.B. das Acetat, als Fungizide zu verwenden (DE-1 164 152, 1 173 722). Ihre Wirkung ist jedoch in manchen Fällen ungenügend. Es ist ferner bekannt, Acrylsäurederivate, z.B. den alpha-(2-Benzyloxyphenyl)-beta-methoxyacrylsäuremethylester, als Fungizid zu verwenden (EP-178826). Seine fungizide Wirkung ist unbefriedigend.

Es wurde nun gefunden, daß neue Acrylsäurederivate der Formel

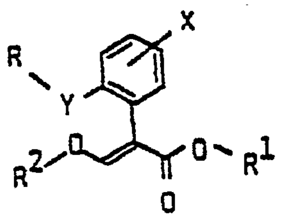

in der $R^1$ und $R^2$ unabhängig voneiander $C_1$-$C_8$-Alkyl, $C_2$-$C_8$-Alkenyl oder $C_3$-$C_8$-Alkinyl bedeuten,
X Wasserstoff, Halogen, $C_1$-$C_4$-Alkyl, $C_1$-$C_4$-Alkoxy, Trifluormethyl, Cyano oder Nitro bedeutet,
Y Ethylen, Ethenylen, Methylenoxy, Oxymethylen, Thiomethylen, Methylenthio oder 0 bedeutet,
R H oder gegebenenfalls durch $C_1$-$C_4$-Alkoxy, Halogen, Cyano-, -$COOR^3$ substituiertes $C_1$-$C_{20}$-Alkyl oder $C_3$-$C_8$-Cycloalkyl, $C_2$-$C_{20}$-Alkenyl oder $C_6$-$C_8$-Cycloalkenyl, $C_3$-$C_{10}$-Alkinyl oder $C_8$-Cycloalkinyl bedeutet und
$R^3$ die gleichen Bedeutungen wie $R^1$ hat und mit $R^1$ gleich oder verschieden ist, außer den Verbindungen, in denen R–Y– den Vinylrest bedeutet und den Verbindungen, in den Y Ethylen oder Sauerstoff und R Wasserstoff, $C_1$–$C_4$–Alkyl, $C_3$–$C_4$–Alkenyl oder Cyclohexyl bedeutet, eine ausgezeichnete fungizide Wirkung haben.

Die in der allgemeinen Formel angeführten Reste können beispielsweise folgende Bedeutung haben:
$R^1$ bzw. $R^2$ $C_1$-$C_8$-Alkyl (z.B. Methyl, Ethyl, Isopropyl, n-Propyl, n-Butyl, sec.-Butyl, tert.-Butyl, iso-Butyl, sec.-Pentyl, n-Hexyl, 2-Ethyl-n-hexyl, n-Octyl), $C_2$-$C_8$-Alkenyl (z.B. Vinyl, Allyl, 1-Methyl-2-propenyl, 3-Methylbutenyl, 3-Methyl-2-butenyl, 2-Methyl-3-butenyl, 4-Octenyl) $C_3$-$C_8$-Alkinyl (z.B. Propargyl, 3-Butinyl, 2-Methylbutinyl-2, 4-Octinyl),
X Wasserstoff, Halogen (z.B. Fluor, Chlor, Brom). $C_1$-$C_4$-Alkoxy (z.B. Methoxy, n-Butoxy), $C_1$-$C_4$-Alkyl (Methyl, Ethyl, Propyl, Butyl) Trifluormethyl, Cyano oder $NO_2$,
Y Ethylen (-$CH_2$-$CH_2$-), Ethenylen (-CH=CH-), Methylenoxy (-$CH_2$-O), Oxymethylen (-O-$CH_2$-), Thiomethylen (-S-$CH_2$-), Methylenthio -($CH_2$-S-) oder -O- bedeutet,
R H oder gegebenenfalls durch $C_1$-$C_4$-Alkoxy, Halogen, Cyano, $COOR^1$ substituiertes $C_1$-$C_{18}$-Alkyl (z.B. Methyl, Ethyl, n-Pentyl, n-Decyl, n-Pentadecyl, n-Octadecyl, n-Eicosanyl, Methoxyethyl, Butoxyethyl, Methoxycarbonylethyl) oder $C_3$-$C_8$-Cycloalkyl (z.B. Cyclopropyl, gem. Dichlorcyclopropyl, Cyclohexyl, Cyclooctyl), $C_2$-$C_{20}$-Alkenyl (z.B. Allyl, 2-Butenyl, 2,6-Dimethyl-2,6-octadienyl) oder $C_6$-$C_8$-Cycloalkenyl (z.B. Cyclohexenyl, Cyclooctadienyl), $C_3$-$C_{10}$-Alkinyl (z.B. Propargyl, 2-Butinyl, Decinyl-10) oder $C_8$-Cycloalkinyl (z.B. Cyclooctinyl).

Die neuen Verbindungen können gegebenenfalls Doppelbindungen und chirale Zentren enthalten. Von der vorliegenden Erfindung werden sowohl die Doppelbindungsisomeren als auch gegebenenfalls Enantiomere und Diastereomere erfaßt. Für die Anwendung als Fungizide können sowohl die einzelnen Isomere als auch ihre bei der Herstellung entstehenden Mischungen verwendet werden.

Die neuen Verbindungen können beispielsweise nach folgendem Verfahren hergestellt werden:
2-Methylphenylessigsäureester der allgemeinen Formel

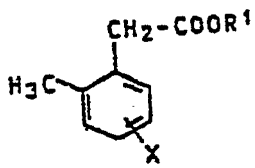

werden nach Wislicenus (Liebigs Annalen 424, 215 (1921) und Liebigs Annalen 413, 206 (1917)) mit Ameisensäuremethylester und Natriumhydrid in einem inerten Lösungsmittel zur Reaktion gebracht. Die so erhaltenen Verbindungen der allgemeinen Formel

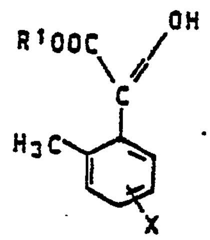

werden mit einem Alkylierungsmittel in Gegenwart einer Base in einem Lösungsmittel (z.B. Aceton) zu V-(2-Methylphenyl)-β-alkoxyacrylsäureestern umgesetzt,

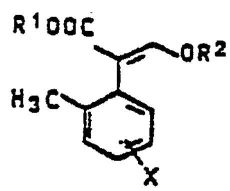

in denen $R^1$, $R^2$ und X die obengenannten Bedeutungen haben.

Die Bromierung dieser Verbindung mit N-Bromsuccinimid (Horner, Winkelmann, Angew. Chemie 71, 349 (1959) führt zu V-(2-Brommethylphenyl)-β-alkoxy-acrylsäureestern, die mit Phosphorigsäuretrialkylestern zu Phosphonsäureestern der allgemeinen Formel reagieren:

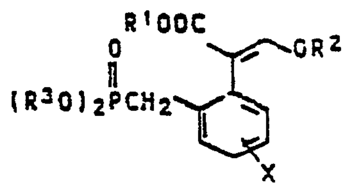

in der $R^1$, $R^2$ und X die obengenannten Bedeutungen haben und $R^3$ für $C_1$-$C_8$-Alkyl steht (Houben-Weyl), Methoden der organischen Chemie 12/1, 433 ff (1963)).

Die obengenannten Phosphonsäureester werden mit gegebenenfalls substituierten Alkyl-, Alkenyl oder Alkinylaldehyden umgesetzt:

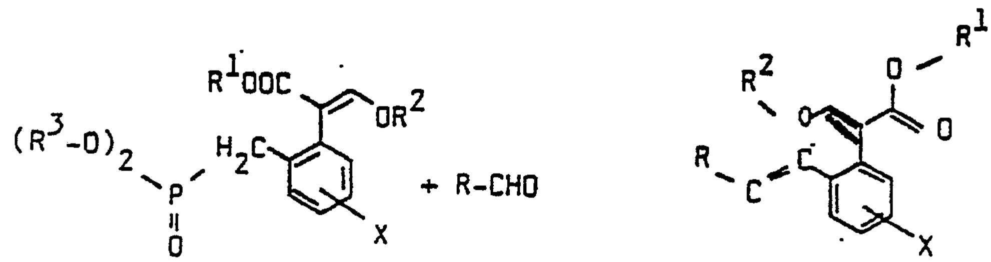

Die so erhaltenen ungesättigten Derivate lassen sich entweder katalytisch mit Wasserstoff (vgl. Houben-Weyl, Methoden der organischen Chemie V/2b, 264-267, (1981)) oder mit Diimin (vgl. Houben-Weyl, Methoden der organischen Chemie IV/1c, 580 und E.E. van Tamelen, R.S. Dewey, M.F. Lease - W.H. Pirkle, JACS 83, 4302 (1961)) selektiv zu neuen Alkoxyacrylsäurederivaten reduzieren:

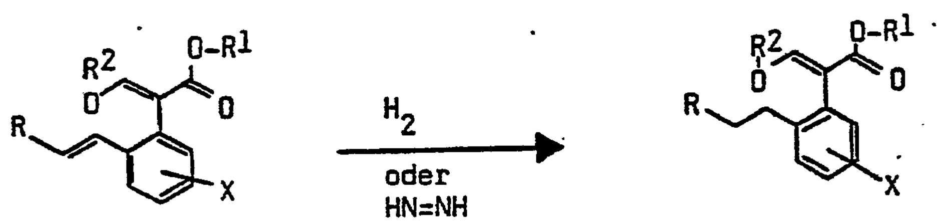

Die als Vorstufe erhaltenen Brommethylphenylalkoxyacrylsäureester können mit Alkoholaten oder Thiolaten in geeigneten Lösungsmitteln (z.B. Tetrahydrofuran, Dimethylformamid, Dimethylsufoxid, Thiolen, Diethylether, Toluol, Benzol, Xylol, Methyl-tert.-butylether) zu den entsprechenden Alkoxy- oder Alkylthioderivaten umgesetzt werden

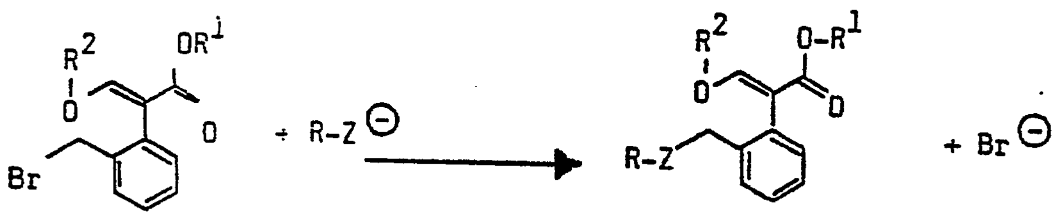

wobei Z O oder S bedeutet.

Ein weiteres Herstellungsverfahren ist z.B. das folgende: Durch den Substituenten R alkylierte 2-Hydroxyphenylessigester können nach der oben erwähnten Vorschrift an der Methylengruppe des Essigesterrestes formyliert und anschließend alkyliert werden.

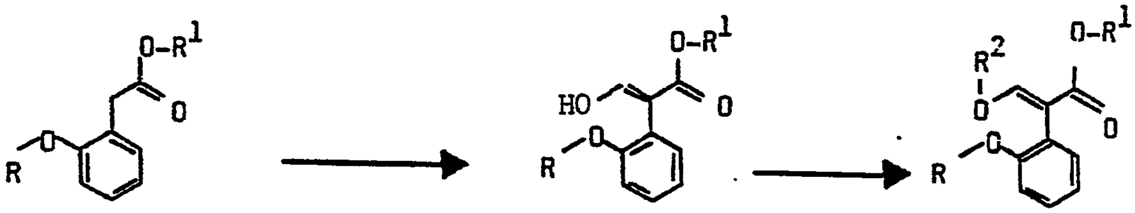

Die nachstehenden Vorschriften erläutern die Herstellung der Ausgangsverbindungen:

Vorschrift A

α-(2-Methylphenyl)-β-methoxy-acrylsäuremethylester

16,5 g 2-Methylphenylessigsäuremethylester werden in 10 ml Ameisensäuremethylester gelöst und langsam zu einer Suspension aus 3 g Natriumhydrid in 150 ml abs. Ether getropft. Nach 4 h Rückfluß wird mit verdünnter HCl angesäuert, die org. Phase abgetrennt, mit Wasser gewaschen, über $MgSO_4$ getrocknet und eingeengt. Man erhält 13,8 g eines hellgelben Öles (α-Formyl-(2-methylphenyl)essigsäuremethylester), das zusammen mit 5,8 ml Dimethylsulfat, 10,9 g Kaliumcarbonat und 70 ml Aceton 1 h am Rückfluß gekocht wird. Nach dem Filtrieren und Einengen wird in Ether aufgenommen, dann mit verdünntem wäßrigem Ammoniak und mehrfach mit Wasser gewaschen. Nach Abziehen des Ethers erhält man 11,3 g rohen α-(2-Methylphenyl)-β-methoxyacrylatsäuremethylester ($KP_{0.05}$ 102-108°C).

| | | | |
|---|---|---|---|
| NMR in $CDCl_3$: | 7,63 | s | 1H |
| | 7,16–7,36 | bs | 4H |
| | 3,64 | s | 3H |
| | 3,73 | s | 3H |
| | 2,16 | s | 3H |

Vorschrift B

α-(2-Brommethylphenyl)-β-methoxyacrylsäuremethylester

20,6 g des nach Vorschrift A erhaltenen α-(2-Methylphenyl)-β-methoxyacrylsäuremethylesters, 17,65 g N-Bromsuccinimid, 0,2 g Azodiisobutyronitril und 150 ml $CCl_4$ werden langsam auf 90°C erhitzt. Man erhält bei dieser Temperatur bis alles Succinimid auf dem Lösungsmittel schwimmt. Nach Filtration wird eingeengt, das verbleibende Öl in etwa 5 ml Aceton gelöst und mit n-Hexan zur Kristallisation gebracht. Man erhält 27,5 g farblose Kristalle vom Fp. 86-87°C.

Vorschrift C

2-(β-Methoxy-α-methoxycarbonyl-vinyl)benzylphosphonsäuredimethylester

28,5 g α-(2-Brommethylphenyl)-β-methoxyacrylsäuremethylester werden mit 11,8 ml Phosphorigsäuretrimethylester und 6,5 ml Toluol eine Stunde am Rückfluß gekocht. Das Reaktionsgemisch wird vorsichtig im Vakuum eingeengt, das verbleibende Öl wird nach Lösen in 5 ml Ether mit n-Hexan zur Kristallisation gebracht. Man erhält 27,3 g farblose Kristalle vom Fp. 94°C.

Vorschrift D

2-n-Butoxyphenylessigsäuremethylester

16,6 g 2-Hydroxyphenylessigsäuremethylester werden in 100 ml Methanol gelöst und mit 15 g Kaliumcarbonat sowie 18 g n-Butylbromid versetzt. Nach 24stündigem Sieden unter Rückfluß wird die filtrierte Lösung im Vakuum eingeengt. Das erhaltene Öl wird in Diethylether aufgenommen und mit $NaHCO_3$ und $H_2O$ gewaschen. Nach Trocknen über Natriumsulfat und Entfernen des Lösungsmittels erhält man 14,5 g (65 %) eines gelben Öls, welches durch Destillation gereinigt werden kann.

NMR in $CDCl_3$: m 7,15 2H; m 6,85 2H; t 3,95 $-CH_2-$; s 3,65 $O-CH_3$;
s 3,62 $-CH_2-$; m 1,75 $-CH_2-$; m 1,48 $-CH_2-$; t 0,95 $-CH_3$

Vorschrift E

α-Formyl-(2-butoxyphenyl)essigsäuremethylester

11,1 g des nach Vorschrift D erhaltenen Produktes werden in 50 ml Ameisensäuremethylester gelöst und bei 0°C langsam 3,5 g Natriummethylat zugegeben. Man läßt auf Raumtemperatur erwärmen und entfernt im Vakuum den größten Teil des Ameisensäuremethylesters. Der Rest wird auf kalte 2 N NaOH gegossen und mit 30 ml Methyl-ter.-butylether extrahiert. Nach Ansäuern der wäßrigen Phase wird der α-Formyl-(2-butoxyphenyl)essigsäuremethylester mit Dichlormethan extrahiert. Nach Ansäuern der wäßrigen Phase wird der α-Formyl-(2-butoxyphenyl)essigsäuremethylester mit Dichlormethan extrahiert. Nach Trocknen der org. Phase über $Na_2SO_4$ erhält man 10,2 g (82 %) reines Produkt.

Beispiel 1

α-(2-(2,6-Dimethyloctanyl-8-oxy)phenyl)-β-methoxyacrylsäuremethylester (Verbindung 2)

3,2 g α-(2-(2,6-Dimethylocta-2,6-dien-yl-8-oxy)-phenyl)-β-methoxyacrylsäuremethylester werden in 70 ml Methanol gelöst und 0,2 g Palladium auf Aktivkohle zugegeben. Bei Raumtemperatur wird unter Rühren Wasserstoff durch die Suspension gegeben bis kein Gas mehr aufgenommen wird. Die Lsg. wird vom Katalysator getrennt und die verbleibende Lsg. eingedampft. Man erhält 3,1 g eines Öls.

Beispiel 2

α-(2-n-Butoxyphenyl)-3-methoxyacrylsäuremethylester (Verbindung 7)

10 g α-Formyl-(2-butoxyphenyl)essigsäuremethylester werden in 100 ml Aceton gelöst, 15 g Kaliumcarbonat zugefügt und langsam unter Rühren mit der äquimolaren Menge Dimethylsulfat versetzt. Nach 12stündigem Kochen unter Rückfluß werden 50 ml $H_2O$ zugesetzt und vorsichtig 30 ml konz. $NH_3$-Lsg. Nach 2stündigem Rühren werden ca. 100 ml Lsg. abgezogen, auf Eis gegossen und mit Dichlormethan extrahiert. Nach Trocknen der org. Phase erhält man 8,7 g Öl.

Beispiel 3

1,44 g NaH (80 %ig) werden unter Stickstoff in 20 ml Tetrahydrofuran (THF) vorgelegt. 15 g Phosphonat aus Vorschrift C und 5,4 g Cyclohexylaldehyd werden in 100 ml THF gelöst und unter Rühren langsam zum NaH zugetropft. Die Reaktionsmischung wurde 10 Stdn. nachrühren lassen und anschließend 200 ml $H_2O$ zugetropft. Nach Extraktion mit Dichlormethan wurde die org. Phase getrocknet über $Na_2SO_4$ und im Vakuum eingedampft. Der verbleibende Rückstand wurde in Toluol gelöst und über Kieselgel filtriert. Es werden 9,3 g eines Harzes (Verbindung 10) erhalten.

Beispiel 4

α-(2-Butoxymethylphenyl)-β-methoxyacrylsäuremethylester (Verbindung 32)

7,4 g n-Butanol werden in 50 ml Dimethylformamid gelöst und 2,4 g Natriumhydrid hinzugefügt. Die Lösung wird leicht erwärmt, bis alles reagiert hat. Diese Lösung wird bei -20°C langsam zu 28,4 g α-(2-Brommethylphenyl)-β-methoxyacrylsäuremethylester in 150 ml THF getropft. Nachdem die Brommethylverbindung nicht mehr dünnschichtchromatographisch nachgewiesen werden kann, werden 100 g Eis und 100 ml gesättigte NaCl-Lösung zugegeben. Durch Extraktioin mit Methylenchlorid erhält man 23 g eines Öls, das chromatographiert wird. Es werden 10 g eines Harzes nach Elution mit Essigester/Hexan erhalten.

In entsprechender Weise lassen sich folgende Verbindungen herstellen:

| Nr. | $R^1$ | $R^2$ | (R-Y-Aromat) | X | R | NMR ($CDCl_3$; ppm) |
|---|---|---|---|---|---|---|
| 1 | $CH_3$ | $CH_3$ | $-CH_2-O-$ | H | 2,6-Dimethyl-2,6-heptadienyl | s 7,43 1H; m 7,6–6,8 4H; t 5,4 1H; s 506; d 4,5; s 3,73; s 3,6; m 2,3; m 1,7; m 1,6 |
| 2 | $CH_3$ | $CH_3$ | $-CH_2-O-$ | H | 2,6-Dimethylheptyl | s 7,42 1H; m 7,6–6,8 4H; m 3,9 2H; s 3,8 5H; s 3,63 3H; m 0,7–2,25 |
| 3 | $CH_3$ | $CH_3$ | $-CH_2-O-$ | H | 2,6,10-Trimethyl-2,6,10-undecyltrienyl | m 7,5–6,7 5H; s 5,3 1H; s 5,0 2 H; s 3,8 3H; s 3,73 2H; s 3,70 3H; m 1,7–2,1 |
| 4 | $CH_3$ | $CH_3$ | $-CH_2-O-$ | H | 2,6,10,14-Tetramethyl-2,6,10,14-pentadecatetraenyl | m 7,5–6,7 5H; t 5,4 1H; s 5,01 1H; s 3,8 3H; s 3,65 3H; s 3,6 5H; m 1,5–2,0 |
| 5 | $CH_3$ | $CH_3$ | $-CH_2-O-$ | H | n-$C_{20}H_{41}$ | |
| 6 | $CH_3$ | $CH_3$ | $-CH_2-O-$ | H | n-$C_{10}H_{21}$ | |
| 10 | $CH_3$ | $CH_3$ | $-CH=CH-$ | H | cyclo $C_6H_{11}$ | s 3,69 3H s 7,58 1H; m 7,51 1H; m 7,18 3H d 6,25 1H; m 6,05 1H; s 3,78 3H; s 3,65 1H; m 0,8–2,2 10H |

| Nr. | $R^1$ | $R^2$ | (R-Y-Aromat) | X | R | NMR ($CDCl_3$; ppm) |
|---|---|---|---|---|---|---|
| 11 | $CH_3$ | $CH_3$ | $-CH=CH-$ | H | cyclo $C_5H_9$ | |
| 12 | $CH_3$ | $CH_3$ | $-CH=CH-$ | H | cyclo $C_7H_{13}$ | |
| 13 | $CH_3$ | $CH_3$ | $-CH=CH-$ | H | cyclo $C_8H_{15}$ | |
| 14 | $CH_3$ | $CH_3$ | $-CH=CH-$ | H | 2,6-Dimethyl-2,6-hepta-dienyl-7- | m 7,60 1H; s 7,55 1H; m 7,3 – 6,85 4H; m 6,35 1H; m 5,95 1H; m 5,1 1H; s 3,78 3H; s 3,66 1H; m 2,35 – 2,0 4H; d 1,85 3H; s 1,7 3H; s 1,6 3H |
| 15 | $CH_3$ | $CH_3$ | $-CH=CH-$ | H | $CH_3$ | |
| 16 | $CH_3$ | $CH_3$ | $-CH=CH-$ | H | $C_2H_5$ | |
| 17 | $CH_3$ | $CH_3$ | $-CH=CH-$ | H | $C_3H_7$ | |
| 18 | $CH_3$ | $CH_3$ | $-CH=CH-$ | H | iso $C_3H_7$ | |
| 19 | $CH_3$ | $CH_3$ | $-CH=CH-$ | H | $C_4H_9$ | |
| 20 | $CH_3$ | $CH_3$ | $-CH=CH-$ | H | tert. $C_4H_9$ | |
| 21 | $CH_3$ | $CH_3$ | $-CH=CH-$ | H | n-$C_{10}H_{21}$ | |
| 22 | $CH_3$ | $CH_3$ | $-CH=CH-$ | H | n-$C_{20}H_{41}$ | |
| 23 | $CH_3$ | $CH_3$ | $-CH=CH-$ | H | Allyl | |
| 24 | $CH_3$ | $CH_3$ | $-CH=CH-$ | H | 1-Methyl-2-propenyl | |
| 25 | $CH_3$ | $CH_3$ | $-CH=CH-$ | H | 1-Methyl-2-propinyl | |
| 26 | $CH_3$ | $CH_3$ | $-CH=CH-$ | H | 2-Propinyl | |
| 29 | $CH_3$ | $CH_3$ | $-CH=CH-$ | H | n-Dodecyl | |

| Nr. | $R^1$ | $R^2$ | (R-Y-Aromat) | X | R | NMR ($CDCl_3$; ppm) |
|---|---|---|---|---|---|---|
| 30 | $CH_3$ | $CH_3$ | $-O-CH_2-$ | H | Methyl | |
| 31 | $CH_3$ | $CH_3$ | $-O-CH_2-$ | H | Ethyl | |
| 32 | $CH_3$ | $CH_3$ | $-O-CH_2-$ | H | n-Butyl | |
| 33 | $CH_3$ | $CH_3$ | $-O-CH_2-$ | H | n-Decyl | |
| 34 | $CH_3$ | $CH_3$ | $-O-CH_2-$ | H | 1-Methyl-2-propenyl | |
| 35 | $CH_3$ | $CH_3$ | $-O-CH_2-$ | H | 2-Propinyl | |
| 36 | $CH_3$ | $CH_3$ | $-O-CH_2-$ | H | 2-Propenyl | |
| 37 | $CH_3$ | $CH_3$ | $-O-CH_2-$ | H | cyclohexyl | |
| 38 | $CH_3$ | $CH_3$ | $-O-CH_2-$ | H | cycloheptyl | |
| 39 | $CH_3$ | $CH_3$ | $-O-CH_2-$ | H | cyclopentyl | |
| 40 | $CH_3$ | $CH_3$ | $-O-CH_2-$ | H | cyclohexen | |
| 41 | $CH_3$ | $CH_3$ | $-O-CH_2-$ | H | Methoxyethyl | |
| 42 | $CH_3$ | $CH_3$ | $-O-CH_2-$ | H | Butoxyethyl | |
| 43 | $CH_3$ | $CH_3$ | $-O-CH_2-$ | H | Methoxycarbonylethyl | |
| 44 | $CH_3$ | $CH_3$ | $-O-CH_2-$ | H | 4-Chlorbutyl | |
| 45 | $CH_3$ | $CH_3$ | $-CH_2-O-$ | H | 4-Chlorpropyl | |
| 46 | $CH_3$ | $CH_3$ | $-CH_2-O-$ | H | 4-Brombutyl | |
| 47 | $CH_3$ | Allyl | $-CH=CH-$ | H | cyclo $C_6H_{11}$ | |
| 48 | $CH_3$ | Allyl | $-CH=CH-$ | H | $CH_3$ | |
| 49 | $CH_3$ | Allyl | $-CH=CH-$ | H | n-$C_4H_9$ | |
| 50 | $CH_3$ | Propargyl | $-CH=CH-$ | H | cyclo $C_4H_{11}$ | |
| 51 | $CH_3$ | Propargyl | $-CH=CH-$ | H | $CH_3$ | |
| 52 | $CH_3$ | Propargyl | $-CH=CH-$ | H | n-$C_4H_9$ | |
| 53 | $CH_3$ | Propargyl | $-CH=CH-$ | H | 1-Methyl-2-propenyl | |

| Nr. | $R^1$ | $R^2$ | (R-Y-Aromat) | X | R | NMR ($CDCl_3$; ppm) |
|---|---|---|---|---|---|---|
| 56 | $CH_3$ | Propargyl | $-CH_2-O-$ | H | 1-Methyl-2-propenyl | |
| 57 | $CH_3$ | Propargyl | $-CH_2-O-$ | H | Cyclohexyl | |
| 58 | $CH_3$ | Allyl | $-CH_2-O-$ | H | Cyclohexyl | |
| 59 | $CH_3$ | Allyl | $-CH_2-O-$ | H | 3-Butenyl | |
| 62 | $CH_3$ | Allyl | $-HC=CH-$ | 4F | Cyclohexyl | |
| 63 | $CH_3$ | Allyl | $-HC=CH-$ | 4F | iso-Propyl | |
| 66 | $C_2H_5$ | $CH_3$ | $-O-CH_2-$ | H | Cyclohexyl | |
| 67 | $n-C_4H_9$ | $CH_3$ | $-O-CH_2-$ | H | Cyclohexyl | |
| 71 | $CH_3$ | $CH_3$ | $-S-CH_2$ | H | $CH_3$ | |
| 72 | $CH_3$ | $CH_3$ | $-S-CH_2$ | H | $C_4H_9$ | |
| 73 | $CH_3$ | $CH_3$ | $-S-CH_2$ | H | $C_{10}H_{21}$ | |
| 74 | $CH_3$ | $CH_3$ | $-S-CH_2$ | H | Cyclohexyl | |
| 75 | $CH_3$ | $CH_3$ | $-S-CH_2$ | H | 1-Methyl-2-propenyl- | |
| 77 | $CH_3$ | $CH_3$ | $-CH_2-CH_2$ | H | 1-Ethylpentyl | s 7,6 1H; m 7,4–7,0 4H; s 3,90 3H s 3,8 3H; m 2,4–2,3 2H; m 1,6–0,7 17H |

| Nr. | $R^1$ | $R^2$ | (R-Y-Aromat) | X | R | NMR ($CDCl_3$; ppm) |
|---|---|---|---|---|---|---|
| 78 | $CH_3$ | $CH_3$ | $-CH_2-CH_2$ | H | 2,6-Dimethylheptyl | s 7,6 1H; m 7,3–7,0 4H; s 3,9 3H; m 2,5–2,35 2H; m 1,6–0,7 20H |
| 79 | $CH_3$ | $CH_3$ | $-CH=CH-$ | H | 1-Ethyl-pentyl | s 7,6 1H; m 7,65–7,1 4H; d 6,3 2H; m 5,95–5,8 1H; s 3,8 3H; s 3,7 3H; m 2,1–0,8 15H |
| 80 | $CH_3$ | $CH_3$ | $-CH=CH-$ | H | 2,6-Dimethylheptyl | s 7,6 1H; m 7,65–6,8 5H; m 6,25–6,15 1H; m 6,0–5,9 1H; m 5,2–5,0 1H; s 3,8 3H; s 3,7 3H; m 2,3–1,45 13H |
| 81 | $CH_3$ | $CH_3$ | $-CH=CH-$ | H | n-Heptyl | s 7,6 1H; m 7,6–7,0 4H; m 6,4–6,0 2H; s 3,8 3H; s 3,7 3H; m 2,25–2,15 2H; m 1,6–0,8 13H |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Septoria nodorum an Weizen,
Botrytis cinerea (Grauschimmel) an Erdbeeren, Reben,

Cercospora arachidicola an Erdnüssen,
Pseudocercosporella herpotrichoides an Weizen, Gerste,
Pyricularia oryzae an Reis,
Hemileia vastatrix an Kaffee,
Alternaria solani an Kartoffeln, Tomaten,
Sclerotium rolfsii an Erdnüssen und Rasen-Arten,
Fusarium- und Verticillium-Arten an verschiedenen Pflanzen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff. Die Aufwandmengen liegen je nach Art des gewünschten Effektes zwischen 0,02 und 3 kg Wirkstoff oder mehr je ha.

Die neuen Verbindungen können auch im Materialschutz eingesetzt werden. Bei der Anwendung der Wirkstoffe im Materialschutz u.a. zur Bekämpfung holzzerstörender Pilze wie Coniophora puteana und Polystictus versicolor eingesetzt werden. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 2 mit 10 Gew.-Teilen N-Methyl-α-pyrrolidon und erhält eine Lösung, die zur Anwendung in Form kleinster Tropfen geeignet ist.

III. 20 Gew.-Teile der Verbindung Nr. 17 werden in einer Mischung gelöst, die aus 40 Gew.-Teilen cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 2 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

VI. 3 Gew.-Teile der Verbindung Nr. 17 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 1 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 2 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 3 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder

auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:
Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N'-propylen-bis-dithiocarbamat),
Zink-(N,N'-propylen-bis-dithiocarbamat),
N,N'-Polypropylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;
5-Nitro-isophthalsäure-di-isopropylester
heterocyclische Substanzen, wie
2-Heptadecyl-2-imidazolin-acetat,
2,4-Dichlor-6-(o-chloranilino)-s-triazin,
0,0-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N', N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan ,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N'-imidazol-yl-harnstoff,
1-(4-Chlorphenoxy)-3,3-dimethyl-1-(1H-1,2,4-triazol-1-yl)-2-butanon,

1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol.

Für die folgenden Versuche wurde als Vergleich die bekannten Wirkstoffe N-Tridecyl-2,6-dimethylmorpholin (A), sein Acetat (B) und der α-(2-Benzyloxyphenyl)-β-methoxyacrylsäuremethylester (C) verwendet.

Anwendungsbeispiel 1

Wirksamkeit gegen Weizenmehltau

Blätter von in Töpfen gewachsenen Weizenkeimlingen der Sorte "Frühgold" werden mit wäßriger Spritzbrühe, die 80 % Wirkstoff und 20 % Emulgiermittel in der Trockensubstanz enthält, besprüht und 24 Stunden nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80 % relativer Luftfeuchtigkeit aufgestellt. Nach 7 Tagen wird das Ausmaß der Mehltauentwicklung ermittelt.

Das Ergebnis des Versuches zeigt, daß die Wirkstoffe Nr. 1, 2 und 3 bei der Anwendung als 0,025 und 0,006 %ige (Gew.%) Spritzbrühe eine bessere fungizide Wirkung (90 %) zeigen als der bekannte Wirkstoff A und B (70 %).

Anwendungsbeispiel 2

Wirksamkeit gegen Weizenbraunrost

Blätter von in Töpfen gewachsenen Weizensämlingen der Sorte "Frühgold" werden mit Sporen des Braunrostes (Puccinia recondita) bestäubt. Danach werden die Töpfe für 24 Stunden bei 20 bis 22°C in eine Kammer mit hoher Luftfeuchtigkeit (90 bis 95 %) gestellt. Während dieser Zeit keimen die Sporen aus und die Keimschläuche dringen in das Blattgewebe ein. Die infizierten Pflanzen werden anschließend mit wäßrigen Spritzbrühen, die 80 % Wirkstoff und 20 % Emulgiermittel in der Trockensubstanz enthalten, tropfnaß gespritzt. Nach dem Antrocknen des Spritzbelages werden die Versuchspflanzen im Gewächshaus bei Temperaturen zwischen 20 und 22°C und 65 bis 70 % relativer Luftfeuchte aufgestellt. Nach 8 Tagen wird das Ausmaß der Rostpilzentwicklung auf den Blättern ermittelt.

Das Ergebnis des Versuches zeigt, daß bei der Anwendung als 0,025 oder 0,006 %ige Spritzbrühe beispielsweise die Verbindungen 1, 2 und 3 eine bessere fungizide Wirkung (100 %) haben als die bekannten Wirkstoffe A und C (70 %).

Anwendungsbeispiel 3

Wirksamkeit gegen Plasmopara viticola

Blätter von Topfreben der Sorte "Müller-Thurgau" werden mit wäßriger Spritzbrühe, die 80 % Wirkstoff und 20 % Emulgiermittel in der Trockensubstanz enthält, besprüht. Um die Wirkungsdauer der Wirkstoffe beurteilen zu können, werden die Pflanzen nach dem Antrocknen des Spritzbelages 10 Tage im Gewächshaus aufgestellt. Erst dann werden die Blätter mit einer Zoosporenaufschwemmung von Plasmopara viticola infiziert. Danach werden die Reben zunächst für 16 Stunden in einer wasserdampfgesättigten Kammer bei 24°C und anschließend für 8 Tage in einem Gewächshaus mit Temperaturen zwischen 20 und 30°C aufgestellt. Nach dieser Zeit werden die Pflanzen zur Beschleunigung des Sporangienträgerausbruches abermals für 16 Stunden in der feuchten Kammer aufgestellt. Dann erfolgt die Beurteilung des Ausmaßes des Pilzausbruches auf den Blattunterseiten.

Das Ergebnis des Versuches zeigt, daß bei der Anwendung als 0,05 %ige Spritzbrühe beispielsweise die Verbindungen 1, 2, 3 und 4 eine gute fungizide Wirkung zeigen (100 %).

Anwendungsbeispiel 4

Wirksamkeit gegen Septoria nodorum

Blätter von in Töpfen gewachsenen Weizenkeimlingen der Sorte "Jubilar" werden mit wäßriger Spritzbrühe, die 80 % Wirkstoff und 20 % Emulgiermittel in der Trockensubstanz enthält, besprüht, nach dem Antrocknen des Spritzbelages abgeschnitten und in Schalen mit wäßriger Benzimidazollösung (25 ppm) ausgelegt. Dann werden die Blätter mit einer wäßrigen Sporensuspension von Septoria nodorum infiziert und abgedeckt. Nach 7-tägigem Stehen bei 20 bis 22°C wird das Ausmaß der Pilzentwicklung ermittelt.

Das Ergebnis des Versuches zeigt, daß bei der Anwendung als 0,05 %ige Spritzbrühe beispielsweise die Verbindungen 1, 2, 3 und 4 eine gute fungizide Wirkung zeigen (100 %).

**Patentansprüche**

1. Substituierte Acrylsäureester der allgemeinen Formel

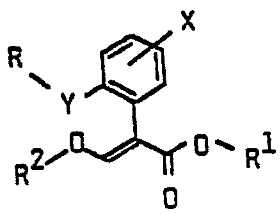

in der $R^1$ und $R^2$ unabhängig voneinander $C_1$-$C_8$-Alkyl, $C_2$-$C_8$-Alkenyl oder $C_3$-$C_8$-Alkinyl bedeuten,
X Wasserstoff, Halogen, $C_1$-$C_4$-Alkyl, $C_1$-$C_4$-Alkoxy, Trifluormethyl, Cyano oder Nitro bedeutet,
Y Ethylen, Ethenylen, Methylenoxy, Oxymethylen, Thiomethylen, Methylenthio oder 0 bedeutet,
R H oder gegebenenfalls durch $C_1$-$C_4$-Alkoxy, Halogen, Cyano, $-COOR^3$ substituiertes $C_1$-$C_{20}$-Alkyl oder $C_3$-$C_8$-Cycloalkyl, $C_2$-$C_{20}$-Alkenyl oder $C_6$-$C_8$-Cycloalkenyl, $C_3$-$C_{10}$-Alkinyl oder $C_8$-Cycloalkinyl bedeutet und
$R^3$ die gleichen Bedeutungen wie $R^1$ hat und mit $R^1$ gleich oder verschieden ist, außer den Verbindungen, in denen R–Y– den Vinylrest bedeutet und den Verbindungen, in den Y Ethylen oder Sauerstoff und R Wasserstoff, $C_1$–$C_4$–Alkyl, $C_3$–$C_4$–Alkenyl oder Cyclohexyl bedeutet.

2. Fungizid, enthaltend einen Trägerstoff und einen substituierten Acrylsäureester der allgemeinen Formel

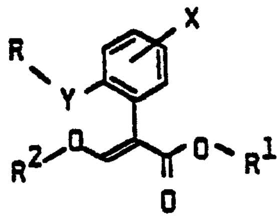

in der $R^1$ und $R^2$ unabhängig voneinander $C_1$-$C_8$-Alkyl, $C_2$-$C_8$-Alkenyl oder $C_3$-$C_8$-Alkinyl bedeuten,
X Wasserstoff, Halogen, $C_1$-$C_4$-Alkyl, $C_1$-$C_4$-Alkoxy, Trifluormethyl, Cyano oder Nitro bedeutet,
Y Ethylen, Ethenylen, Methylenoxy, Oxymethylen, Thiomethylen, Methylenthio oder 0 bedeutet,
R H oder gegebenenfalls durch $C_1$-$C_4$-Alkoxy, Halogen, Cyano, $-COOR^3$ substituiertes $C_1$-$C_{20}$-Alkyl oder $C_3$-$C_8$-Cycloalkyl, $C_2$-$C_{20}$-Alkenyl oder $C_6$-$C_8$-Cycloalkenyl, $C_3$-$C_{10}$-Alkinyl oder $C_8$-Cycloalkinyl bedeutet und
$R^3$ die gleichen Bedeutungen wie $R^1$ hat und mit $R^1$ gleich oder verschieden ist, außer den Verbindungen, in denen R–Y– den Vinylrest bedeutet und den Verbindungen, in denen Y Ethylen oder Sauerstoff und R Wasserstoff, $C_1$–$C_4$–Alkyl, $C_3$–$C_4$–Alkenyl oder Cyclohexyl bedeutet.

3. Verfahren zur Bekämpfung von Pilzen, dadurch gekennzeichnet, daß man die Pilze oder die von Pilzbefall bedrohten Materialien, Pflanzen, Saatgüter oder den Boden mit einer fungizid wirksamen Menge eines substituierten Acrylsäureesters der allgemeinen Formel

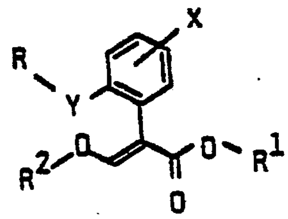

in der $R^1$ und $R^2$ unabhängig voneinander $C_1$-$C_8$-Alkyl, $C_2$-$C_8$-Alkenyl oder $C_3$-$C_8$-Alkinyl bedeuten, X Wasserstoff, Halogen, $C_1$-$C_4$-Alkyl, $C_1$-$C_4$-Alkoxy, Trifluormethyl, Cyano oder Nitro bedeutet, Y Ethylen, Ethenylen, Methylenoxy, Oxymethylen, Thiomethylen, Methylenthio oder 0 bedeutet, R H oder gegebenenfalls durch $C_1$-$C_4$-Alkoxy, Halogen, Cyano, -$COOR^3$ substituiertes $C_1$-$C_{20}$-Alkyl oder $C_3$-$C_8$-Cycloalkyl, $C_2$-$C_{20}$-Alkenyl oder $C_6$-$C_8$-Cycloalkenyl, $C_3$-$C_{10}$-Alkinyl oder $C_8$-Cycloalkinyl bedeutet und $R^3$ die gleichen Bedeutungen wie $R^1$ hat und mit $R^1$ gleich oder verschieden ist, außer den Verbindungen, in denen R–Y– den Vinylrest bedeutet und den Verbindungen, in denen Y Ethylen oder Sauerstoff und R Wasserstoff, $C_1$1–$C_4$–Alkyl, $C_3$–$C_4$–Alkenyl oder Cyclohexyl bedeutet, behandelt.

4. Verbindung der allgemeinen Formel gemäß Anspruch 1, dadurch gekennzeichnet, daß $R^1$ und $R^2$ Methyl, Y $CH_2O$, X H und R 2,6-Dimethyl-2,6-heptadienyl bedeuten.

5. Verbindung der allgemeinen Formel gemäß Anspruch 1, dadurch gekennzeichnet, daß $R^1$ und $R^2$ Methyl, Y $CH_2O$, X H und R 2,6-Dimethylheptyl bedeuten.

6. Verbindung der allgemeinen Formel gemäß Anspruch 1, dadurch gekennzeichnet, daß $R^1$ und $R^2$ Methyl, Y $CH_2O$, X H und R 2,6, 10-Trimethyl-2,6,10-undecyltrienyl bedeuten.

7. Verbindung der allgemeinen Formel gemäß Anspruch 1, dadurch gekennzeichnet, daß $R^1$ und $R^2$ Methyl, Y $CH_2O$, Y H und R 2, 6, 10, 14-Tetramethyl-2, 6, 10, 14-pentadecatetraenyl bedeuten.

## Claims

1. A substituted acrylate of the general formula

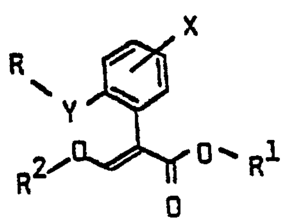

where $R^1$ and $R^2$ are identical or different and each denotes $C_1$–$C_8$-alkyl, $C_2$-$C_8$-alkenyl or $C_3$-$C_8$-alkynyl, X is hydrogen, halogen, $C_1$–$C_4$-alkyl, $C_1$-$C_4$alkoxy, trifluoromethyl, cyano or nitro, Y is ethylene, ethenylene, methyleneoxy, oxymethylene, thiomethylene, methylenethio or oxygen, R is hydrogen or unsubstituted or $C_1$-$C_4$-alkoxy-, halogen-, cyano- or -$COOR^3$-substituted $C_1$-$C_{20}$-alkyl, $C_3$-$C_8$-cycloalkyl, $C_2$–$C_{20}$-alkenyl, $C_6$–$C_8$-cycloalkenyl, $C_3$-$C_{10}$-alkynyl or $C_8$-cycloalkynyl, and $R^3$ has the same meanings as $R^1$ and is identical to or different from $R^1$ but not a compound where R-Y- is vinyl or where Y is ethylene or oxygen and R is hydrogen, $C_1$–$C_4$-alkyl, $C_3$-$C_4$-alkenyl or cyclohexyl.

2. A fungicide containing a carrier and a substituted acrylate of the general formula

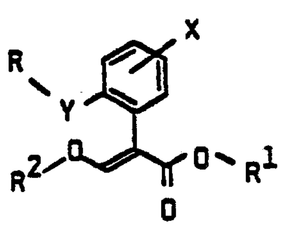

where $R^1$ and $R^2$ are identical or different and each denotes $C_1$–$C_8$-alkyl, $C_2$–$C_8$-alkenyl or $C_3$–$C_8$-alkynyl, X is hydrogen, halogen, $C_1$–$C_4$-alkyl, $C_1$–$C_4$-alkoxy, trifluoromethyl, cyano or nitro, Y is ethylene, ethenylene, methyleneoxy, oxymethylene, thiomethylene, methylenethio or oxygen, R is hydrogen or unsubstituted or $C_1$–$C_4$-alkoxy-, halogen-, cyano- or -$COOR^3$-substituted $C_1$–$C_{20}$-alkyl, $C_3$–$C_8$-cycloalkyl, $C_2$–$C_{20}$-alkenyl, $C_6$–$C_8$-cycloalkenyl, $C_3$–$C_{10}$-alkynyl or $C_8$-cycloalkynyl, and $R^3$ has the

same meanings as $R^1$ and is identical to or different from $R^1$ but not a compound where R-Y- is vinyl or where Y is ethylene or oxygen and R is hydrogen, $C_1$–$C_4$-alkyl, $C_3$–$C_4$-alkenyl or cyclohexyl.

3. A process for controlling fungi, wherein the fungi or the materials, plants, seed or the soil threatened by the fungi or by fungal attack are treated with a fungicidally effective amount of a substituted acrylate of the general formula

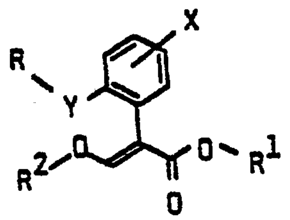

where $R^1$ and $R^2$ are identical or different and each denotes $C_1$–$C_8$-alkyl, $C_2$–$C_8$-alkenyl or $C_2$–$C_8$-alkynyl, X is hydrogen, halogen, $C_1$–$C_4$-alkyl, $C_1$–$C_4$-alkoxy, trifluoromethyl, cyano or nitro, Y is ethylene, ethenylene, methyleneoxy, oxymethylene, thiomethylene, methylenethio or oxygen, R is hydrogen or unsubstituted or $C_1$–$C_4$-alkoxy-, halogen-, cyano- or -$COOR^3$-substituted $C_1$–$C_{20}$-alkyl, $C_3$–$C_8$-cycloalkyl, $C_2$–$C_{20}$-alkenyl, $C_6$–$C_8$-cycloalkenyl, $C_3$–$C_{10}$-alkynyl or $C_8$-cycloalkynyl, and $R^3$ has the same meanings as $R^1$ and is identical to or different from $R^1$ but not a compound where R-Y- is vinyl or where Y is ethylene or oxygen and R is hydrogen, $C_1$–$C_4$-alkyl, $C_3$–$C_4$-alkenyl or cyclohexyl.

4. A compound of the general formula as claimed in claim 1, where $R^1$ and $R^2$ are each methyl, Y is $CH_2O$, X is H and R is 2,6-dimethyl-2,6-heptadienyl.

5. A compound of the general formula as claimed in claim 1, where $R^1$ and $R^2$ are each methyl, Y is $CH_2O$, X is H and R is 2,6-dimethylheptyl.

6. A compound of the general formula as claimed in claim 1, where $R^1$ and $R^2$ are each methyl, Y is $CH_2O$, X is H and R is 2,6,10-trimethyl-2,6,10-undecyltrienyl.

7. A compound of the general formula as claimed in claim 1, where $R^1$ and $R^2$ are each methyl, Y is $CH_2O$, X is H and R is 2,6,10,14-tetramethyl-2, 6,10,14-pentadecatetraenyl.

**Revendications**

1. Esters d'acide acrylique substitués de formule générale

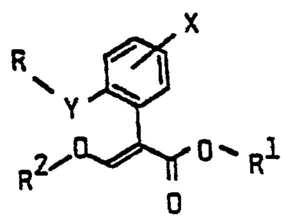

dans laquelle $R^1$ et $R^2$ représentent, indépendamment l'un de l'autre, alkyle en $C_1$–$C_8$, alcényle en $C_2$–$C_8$ ou alcynyle en $C_3$–$C_8$,
X, hydrogène, halogène, alkyle en $C_1$–$C_4$, alcoxy en $C_1$–$C_4$, trifluorométhyle, cyano ou nitro,
Y, éthylène, éthénylène, méthylènoxy, oxyméthylène, thiométhylène, méthylènethio ou O,
R, H ou alkyle en $C_1$–$C_{20}$ ou cycloalkyle en $C_3$–$C_8$, alcényle en $C_2$–$C_{20}$ ou cycloalcényle en $C_6$–$C_8$, alcynyle en $C_3$–$C_{10}$ ou cycloalcynyle en $C_8$, éventuellement substitués par alcoxy en $C_1$–$C_4$, halogène, cyano, -$COOR^3$, et
$R^3$, ayant les mêmes significations que $R^1$ et étant identique à ou différent de $R^1$,
exceptés les composés dans lesquels R-Y- représente le reste vinyle et exceptés les composés dans lesquels Y représente éthylène ou oxygène et R hydrogène, alkyle en $C_1$–$C_4$, alcényle en $C_3$–$C_4$ ou cyclohexyle.

2. Fongicide, contenant un support et un ester d'acide acrylique substitué de formule générale

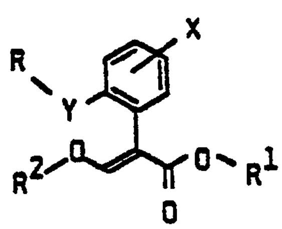

dans laquelle $R^1$ et $R^2$ représentent, indépendamment l'un de l'autre, alkyle en $C_1$–$C_8$, alcényle en $C_2$–$C_8$ ou alcynyle en $C_3$–$C_8$,
X, hydrogène, halogène, alkyle en $C_1$–$C_4$, alcoxy en $C_1$–$C_4$, trifluorométhyle, cyano ou nitro,
Y, éthylène, éthénylène, méthylènoxy, oxyméthylène, thiométhylène, méthylènothio ou O,
R, H ou alkyle en $C_1$–$C_{20}$ ou cycloalkyle en $C_3$–$C_8$, alcényle en $C_2$–$C_{20}$ ou cycloalcényle en $C_6$–$C_8$, alcynyle en $C_3$–$C_{10}$ ou cycloalcynyle en $C_8$, éventuellement substitués par alcoxy en $C_1$–$C_4$, halogène, cyano, -$COOR^3$,
$R^3$, ayant les mêmes significations que $R^1$ et étant identique à ou différent de $R^1$,
exceptés les composés dans lesquels R-Y- représente le reste vinyle et exceptés les composés dans lesquels Y représente éthylène ou oxygène et R hydrogène, alkyle en $C_1$–$C_4$, alcényle en $C_3$–$C_4$ ou cyclohexyle.

3. Procédé de lutte contre les champignons, caractérisé par le fait que l'on traite les champignons ou les matériaux, les plantes, semences ou le sol menacés par l'attaque par les champignons, avec une quantité efficace au point de vue fongicide d'un ester d'acide acrylique substitué de formule générale

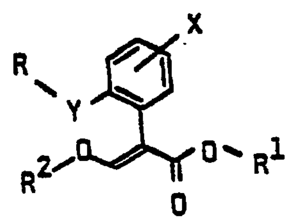

dans laquelle $R^1$ et $R^2$ représentent, indépendamment l'un de l'autre, alkyle en $C_1$–$C_8$, alcényle en $C_2$–$C_8$ ou alcynyle en $C_3$–$C_8$,
X, hydrogène, halogène, alkyle en $C_1$–$C_4$, alcoxy en $C_1$–$C_4$, trifluorométhyle, cyano ou nitro,
Y, éthylène, éthénylène, méthylènoxy, oxyméthylène, thiométhylène, méthylènethio ou O,
R, H ou alkyle en $C_1$–$C_{20}$ ou cycloalkyle en $C_3$–$C_8$, alcényle en $C_2$–$C_{20}$ ou cycloalcényle en $C_6$–$C_8$, alcynyle en $C_3$–$C_{10}$ ou cycloalcynyle en $C_8$, éventuellement substitués par alcoxy en $C_1$–$C_4$, halogène, cyano, -$COOR^3$,
$R^3$, ayant les mêmes significations que $R^1$ et étant identique à ou différent de $R^1$,
exceptés les composés dans lesquels R-Y- représente le reste vinyle et exceptés les composés dans lesquels Y représente éthylène ou oxygène et R hydrogène, alkyle en $C_1$–$C_4$, alcényle en $C_3$–$C_4$ ou cyclohexyle.

4. Composé de formule générale selon la revendication 1, caractérisé par le fait que $R^1$ et $R^2$ représentent méthyle, _Y_, $CH_2O$, _X_, H et _R_, 2,6-diméthyl-2,6-heptadiényle.

5. Composé de formule générale selon la revendication 1, caractérisé par le fait que $R^1$ et $R^2$ représentent méthyle, _Y_, $CH_2O$, _X_, H et _R_, 2,6-diméthylheptyle.

6. Composé de formule générale selon la revendication 1, caractérisé par le fait que $R^1$ et $R^2$ représentent méthyle, _Y_, $CH_2O$, _X_, H et _R_, 2,6,10-triméthyl-2,6,10-undécyltriényle.

7. Composé de formule générale selon la revendication 1, caractérisé par le fait que $R^1$ et $R^2$ représentent méthyle, _Y_, $CH_2O$, _X_, H et _R_, 2,6,10,14-tétraméthyl-2,6,10,14-pentadécatétraényle.